# EUROPÄISCHE PATENTSCHRIFT

(11) **EP 0 692 192 B1**
(45) Veröffentlichungstag und Bekanntmachung des Hinweises auf die Patenterteilung: **15.11.2000**
(21) Anmeldenummer: 95110574.1
(22) Anmeldetag: 06.07.1995
(51) Int. Cl.: A01N 35/02

(54) **Verwendung einer alkoholischen Desinfektionsmittelzubereitung**
Use of an alcoholic disinfecting agent
Utilisation d'une préparation alcoolique d'agent désinfectant

(30) Priorität: 11.07.1994 DE 4424325; 29.01.1995 DE 19502456
(43) Veröffentlichungstag der Anmeldung: 17.01.1996
(73) Patentinhaber: HENKEL-ECOLAB GmbH & CO. OHG, 40589 Düsseldorf (DE)
(72) Erfinder: Widulle, Herbert, Dr., D-22547 Hamburg (DE); Bode, Rüdiger, D-22761 Hamburg (DE); Steinmann, Jürgen, Dr., D-28357 Bremen (DE)

(56) Entgegenhaltungen:
- EP-A- 0 176 720
- EP-A- 0 251 303
- WO-A-95/05737
- DE-A- 4 200 499
- DE-A- 4 241 079
- GB-A- 2 189 146
- CHEMICAL ABSTRACTS, vol. 51, no. 20, 1957 Columbus, Ohio, US; abstract no. 15896c, B.A.LINDENBERG ET AL.: 'The comparative cytolytic power of alcohols, ketones and phenols against bakers' yeast' & J.PHYSIOL. (PARIS), Bd. 49, 1957 Seiten 285-287,
- Römpps Chemie Lexikon, 8. Auflage, Stuttgart 1988, Stichwort: Vergällungsmittel"

## Beschreibung

Die Erfindung betrifft die Verwendung einer alkoholischen Desinfektionsmittel-Zubereitung, die vergleichsweise hohe Mengen an Ethanol und/oder Methanol und außerdem Butanon (Methylethylketon) enthält, zur Haut- und Händedesinfektion unter Inaktivierung unbehüllter Viren und unter Inaktivierung behälter Viren.

Seit vielen Jahren werden im deutschsprachigen Raum für die Hände- und Hautdesinfektion alkoholische Lösungen verwendet. Bei der Händedesinfektion sollen die auf der Haut befindlichen Krankheitserreger abgetötet und damit Infektionsketten von einem Patienten auf den nächsten oder vom Arzt auf einen Patienten unterbrochen werden. In erster Linie sind hier die von außen auf die Haut gelangten Erreger betroffen. Um eine weitere Verbreitung der Erreger zu verhindern, müssen die Hände mit Hilfe eines chemischen Mittels desinfiziert werden.

Obwohl das Risiko der Übertragung unbehüllter Viren wie beispielsweise Polioviren durch Infektionsketten mit Hautberührung seit vielen Jahren bekannt ist, werden immer noch Mittel eingesetzt, die unbehüllte Viren entweder überhaupt nicht oder nur langsam abtöten.

Die oben geschilderten Probleme bei der Haut- und Händedesinfektion unter Abtötung von auf der Haut befindlichen Krankheitserregern betreffen nicht nur unbehüllte, sondern auch behüllte Viren. Das Risiko der Übertragung behüllter Viren wie beispielsweise Hepatitis-Viren, wie Hepatitis B-Viren (HBV) und HI-Viren (human immunodeficiency virus; HIV) ist in der täglichen Arbeit in Kliniken in jüngerer Zeit präsenter denn je. Trotzdem werden in weitem Umfang Desinfektionsmittel-Zubereitungen eingesetzt, die behüllte Viren entweder überhaupt nicht oder nur langsam abtöten.

Aus dem Stand der Technik bekannte Mittel der An, wie sie praktisch verwendet werden, enthalten beispielsweise 30 % n-Propanol, 45 % Isopropanol und 0,2 % Mecetroniumetilsulfat. Ein weiteres in der Praxis verwendetes Mittel enthält 80 % Ethanol und Tetrabromcresol. Alle derzeit praktisch verwendeten Mittel enthalten maximal 80 % Alkohol. Es wurde nämlich angenommen und galt in diesem Bereich der Technik als Dogma, daß Mittel mit Alkoholgehalten über 80 % nicht mehr ausreichend wirken.

In jüngerer Zeit wurde in der EP-A 0 176 720 ein Desinfektionsmittel zur Händedesinfektion beschrieben, das neben mindestens 70 % Ethanol oder Methanol nur noch Wasser, Glycerin und Ricinusöl enthält. In einer Ausführungsform enthält das verwendete Desinfektionsmittel neben Ethanol 4 % Glycerin und 1 % Ricinusöl, d. h. der Gehalt an Ethanol liegt über 80 %. Das in der EP-A 0 176 720 beschriebene Mittel ist wirksam gegen Polioviren, hat aber mehrere Nachteile. Zum einen hinterläßt es nach der Applikation auf der Haut ein unangenehmes Gefühl, das ersichtlich von der Einwirkung der neben Ethanol vorliegenden Komponenten herrührt, zum anderen muß es zur sicheren Inaktivierung unbehüllter Viren wie beispielsweise Polioviren zweimal hintereinander appliziert werden. Wegen der langsamen Wirkung des Mittels gegenüber unbehüllten Viren wie Polioviren empfehlen die Erfinder dieses Mittels bei der praktischen Anwendung eine zweimalige Applikation von je 30 Sekunden Dauer, um Polioviren auf der Haut zu inaktivieren.

Ein weiteres, gegen unbehüllte Viren wie beispielsweise Polioviren wirksames Desinfektionsmittel wird in der EP-A 0 251 303 beschrieben. Das Mittel auf der Basis Ethanol und/oder Propanol ist gegen unbehüllte Viren wirksam, wenn es neben mindestens 70 % Alkohol auch 0,5 bis 5 Gew.-% einer Mono-, Di- oder Tricarbonsäure mit einer Kettenlänge von 2 bis 4 Kohlenstoffatomen oder Sulfaminsäure enthält. Bei Anwendung dieses Desinfektionsmittels sinkt der Virustiter innerhalb von ein bis zwei Minuten um vier Zehnerpotenzen.

Neben der noch immer nicht ausreichenden Wirksamkeit des Mittels ist als Nachteil zu erwähnen, daß dieses einen sehr niedrigen pH-Wert aufweist. Dieser ist auf den vergleichsweisen hohen Anteil an Carbonsäure oder Sulfaminsäure zurückzuführen. Der pH-Wert liegt in den meisten Fällen unter dem natürlichen pH-Wert der Haut. Die Hautverträglichkeit des Desinfektionsmittels wird dadurch in einem Maße verschlechtert, die die Anwendung des Mittels in der Praxis verbietet.

Gleichermaßen für die praktische Anwendung wenig geeignet ist ein Desinfektionsmittel, das in der DE-OS 42 00 499.3 beschrieben wurde. Dieses Desinfektionsmittel auf Alkoholbasis enthält adstringierend wirkende Aluminiumsalze. Durch den Gehalt an Aluminiumsalzen an einem derartigen alkoholischen Händedesinfektionsmittel kann die Haut so stark geschädigt werden, daß eine Applikation nur im Notfall oder Seuchenfall vertretbar ist. Für diese Situation sind jedoch bereits Desinfektionsmittel auf Basis verdünnter Peressigsäure oder Chloramin T beschrieben.

Die WO-A-95/05737 offenbart ein alkoholisches Händedesinfektionsmittel, das als Hautpflegekomponenten Wollwachsalkohole und/oder Cholesterol (Cholesterin) enthält. Als Alkohole werden Ethanol, 1-Propanol und/oder 2-Propanol oder deren Gemische verwendet. Gemäß den Beispielen wird 96 %iges Ethanol, vergällt mit 1 % Mehtylethylketon (2-Butanon), eingesetzt.

Die DE-A-42 41 079 betrifft ein Flächendesinfektionsmittel insbesondere für Kunststoffoberflächen auf Basis kurzkettiger Alkohole. Das Mittel enthält in wäßriger Lösung 20 bis 30 Gew.-% Ethanol und/oder 1-Propanol und/oder 2-Propanol sowie 0,001 bis 0,1 Gew.-% einer freien Säure aus der Gruppe Alkylbenzolsulfonsäuren, Ethercarbonsäuren und Alkanphosphonsäuren. Gemäß den Beispielen wird 94,3 %iges Ethanol, vergällt mit 1 % Methylehtylketon, eingesetzt. Zusätzlich können derartige Mittel noch Peroxide, beispielsweise das Addukt aus Peroxid und Harnstoff, Persessigsäure oder Perphthalsäure enthalten.

Wenn ein Desinfektionsmittel jedoch erst im anerkannten Seuchenfall verwendet wird, müssen im Vorfeld Infektionen infolge nicht ausreichender Inaktivierung unbehüllter Viren wie beispielsweise Polioviren hingenommen werden, wenn nicht auf Produkte zurückgegriffen wird, die in anderer Weise eine ausreichende Desinfektionswirkung gegen unbehüllte Viren erbringen.

Es beseht daher nach wir vor ein großer Bedarf nach Desinfektionsmitteln für die Hände- und Hautdesinfektion, die auch gegen unbehüllte Viren wie beispielsweise Polioviren in kurzer Zeit wirksam sind. Es war Aufgabe der Erfindung, ein Mittel aufzufinden, das mit den bisherigen Verfahren der hygienischen oder chirurgischen Händedesinfektion applizierbar ist, gut hautverträglich ist und auf der Haut als angenehm empfunden wird. Es war eine weitere Aufgabe der Erfindung, ein Desinfektionsmittel aufzufinden, das nach der definierten Versuchsmethodik der Deutschen Gesellschaft für Hygiene und Mikrobiologie (DGHM) bei der chirurgischen Händedesinfektion in fünf Minuten, bevorzugt in drei Minuten, besonders bevorzugt in zwei Minuten, eine mindestens gleichgroße Keimabtötungsrate wie das Referenzverfahren bewirkt und bei der hygienischen Händedesinfetkion in maximal einer Minute, bevorzugt in 30 Sekunden, eine gleiche oder bessere Keimabtötung erzielt wie das Referenzverfahren. Eine weitere Aufgabe der Erfindung war es, ein Desinfektionsmittel aufzufinden, mit dem bei der hygienischen Händedesinfektion eine Inaktivierung von Polioviren in dreißig Sekunden erreicht werden kann.

Eine weitere Aufgabe der Erfindung bestand darin, ein derartiges Mittel aufzufinden, das unter Verwendung preiswert erhältlicher Ausgangssstoffe Bereitgestellt werden kann.

Die genannten Aufgaben werden erfindungsgemäß gelöst durch die Verwendung einer alkoholischen Desinfektionsmittel-Zubereitung mit einem Gehalt an Ethanol und/oder Methanol von mindestens 80 Gew.-% zur Inaktivierung von Viren bei der Hände- und Hautdesinfektion, dadurch gekennzeichnet, daß die Desinfektionsmittelzubereitung Butanon enthält.

Überraschend wurde nämlich gefunden, daß sich mit Butanon, das sonst als Vergällungsmittel in käuflichem Brennspiritus Verwendung findet, die seit langer Zeit angezweifelte, jedenfalls jedoch vergleichsweise geringe Wirksamkeit von reinem Ethanol gegen unbehüllte Viren wie beispielsweise Polioviren signifikant verbessern läßt.

Die Erfindung betrifft die Verwendung einer derartigen alkoholischen Desinfektionsmittel-Zubereitung zur Haut- und Händedesinfektion unter Inaktivierung unbehüllter Viren.

Es besteht nach wie vor ein großer Bedarf nach Desinfektionsmitteln für die Hände- und Hautdesinfektion, die auch gegen behüllte Viren wie beispielsweise Hepatitis-Viren wie Hepatitis B-Viren (HBV) und HI-Viren (human immunodeficiency virus; HIV) in kurzer Zeit wirksam sind. Es war Aufgabe der Erfindung, ein Mittel aufzufinden, das mit den bisherigen Verfahren der hygienischen oder chirurgischen Händedesinfektion applizierbar ist, gut hautverträglich ist und auf der Haut als angenehm empfunden wird. In Ergänzung zur Wirksamkeit der oben angegebenen alkoholischen Desinfektionsmittel-Zubereitung gegen unbehüllte Viren wie beispielsweise Polio-Viren wurde nun überraschend gefunden, daß die Mittel eine nicht zu erwartende Aktivität in der Inaktivierung behüllter Viren wie beispielsweise Hepatitis B-Viren oder HI-Viren Haut- und Händedesinfektion aufweisen. Es konnte daher mit dem erfindungsgemäß zü verwendenden Desinfectionsmittel ein seit langer Zeit bestehender Bedarf erfüllt werden.

Die Erfindung betrifft also die Verwendung einer alkoholischen Desinfektionsmittel-Zubereitung gemäß der oben angegebenen Spezifikation zur Haut- und Händedesinfektion unter Inaktivierung behüllter Viren, insbesondere zur Inaktivierung von Hepatitis B-Viren und HI-Viren.

Die erfindungsgemäß zu verwendende alkoholische Desinfektionsmittel-Zubereitung enthält Ethanol und/oder Methanol in einer Menge von mindestens 80 Gew.-%. Desinfektionsmittel mit derart hohen Alkoholgehalten wirken sehr gut gegen Bakterien und Pilze, was im Hinblick auf den vorstehend beschriebenen Stand der Technik überraschend ist. Überraschenderweise wirken Desinfektionsmittel mit derart hohen Alkoholgehalten aber auch sehr gut gegen behüllte Viren wie beispielsweise HBV-Viren. In einer bevorzugten Ausführungsform enthält die erfindungsgemaß zu verwendende alkoholische Desinfektionsmittel-Zubereitung überwiegend, wenn nicht sogar ausschließlich Ethanol als alkoholische Komponente. Besonders bevorzugt liegt die Konzentration an Ethanol bei einem Wert im Bereich von 85 bis 98 %. Es können jedoch auch mehr oder weniger große Mengen an Methanol oder anderen einwertigen Alkoholen zugegen sein.

Die erfindungsgemäß zu verwendende alkoholische Desinfektionsmittel-Zubereitung enthält als essentiellen Wirkstoff Butanon (Methylethylketon). Wie nachfolgend in den Beispielen gezeigt wird, läßt sich durch den Butanon-Zusatz eine signifikante Verbesserung der Wirksamkeit der Desinfektionsmittel-Zubereitung gegen Polioviren, jedoch auch gegen behüllte Viren wie HBV und HIV erreichen.

Gemäß einer bevorzugten Ausführungsform liegt der Butanon-Gehalt der erfindungsgemäß zu verwendenden alkoholischen Desinfektionsmittel-Zubereitung bei mindestens 0,3 Gew.-%, besonders bevorzugt im Bereich von 0,3 bis 2,5 Gew.-%, mit besonderem Vorteil zwischen 0,8 und 1,3 Gew.-%. In der Praxis zeigt sich nämlich, daß in bezug auf die Reduktion des Titers unbehüllter Viren um mehr als vier Zehnerpotenzen eine Mischung aus 90 % reinem Ethanol und 10 % Wasser erst in einer Minute wirksam ist, während eine Mischung aus 90 % Ethanol, 0,9 % Butanon und zum Rest Wasser den Titer einer identischen Poliosuspension bereits innerhalb von 30 Sekunden um vier bis fünf Zehnerpotenzen senkt.

Gemäß einer weiteren Ausführungsform enthält die erfindungsgemäß zu verwendende alkoholische Desinfektionsmittel-Zubereitung zusätzlich zu den vorstehend genannten Komponenten einen sogenannten Remanenzwirkstoff, bevorzugt aus der Gruppe Chlorhexidin und Benzalkoniumchlorid sowie deren Derivate. In einer weiter bevorzugten Ausführungsform ist der Remanenzwirkstoff Chlorhexidindigluconat. Die Konzentration an Remanenzwirkstoffbeträgt in weiter bevorzugten Ausführungsformen der erfindungsgemäß zu verwendenden Desinfektionsmittel-Zubereitung 0,025 bis 1,0 Gew.-%, wobei Chlorhexidindigluconat-Mengen im vorgenannten Bereich, mit besonderem Vorteil 0,2 % Chlorhexidindigluconat, zu einer besonders guten Wirkung führen. Insbesondere ist die Wirksamkeit innerhalb der ersten dreißig Sekunden des Einwirkens deutlich besser als im Stand der Technik. Da die hygienische Händedesinfektion in der Regel nur 30 Sekunden dauert (vgl. auch DGHM-Test zur hygienischen Händedesinfektion), ist gerade die gesteigerte Wirksamkeit innerhalb der ersten dreißig Sekunden von großer Bedeutung für die Wirkung des Mittels.

In weiter bevorzugten Ausführungsformen der erfindungsgemäß zu verwendenden alkoholischen Desinfektionsmittel-Zubereitung sind zusätzlich als Pflegekomponenten Ester einer langkettigen Fettsäure enthalten. Mit besonderem Vorteil können als Pflegekomponente C₁- bis C₈-Alkylester einer Fettsäure mit 8 bis 18 Kohlenstoffatomen verwendet werden, besonders bevorzugt C₁-bis C₄-Alkylester einer Fettsäure mit 12 bis 18 Kohlenstoffatomen. Isopropylmyristat, Isostearylhexanoat oder Octyloctanoat sind als beispielhafte Verbindungen mit pflegender Wirkung zu nennen, obwohl die Erfindung nicht auf diese beschränkt ist.

In einer weiteren, ebenfalls bevorzugten Ausführungsform enthält die erfindungsgemäß zu verwendende alkoholische Desinfektionsmittel-Zubereitung zusätzlich zu den vorstehenden Komponenten, von denen die alkoholische Komponente und Butanon essentiell, die restlichen Komponenten jedoch optionell sind, ein Feuchthaltemittel. Dieses soll die desinfizierende Wirksamkeit nicht beeinträchtigen, jedoch die Haut mit der notwendigen Feuchtigkeit versorgen und damit pflegend wirken. Dieses Erfordernis ist deswegen wichtig, weil ein Desinfektionsmittel häufig aufgebracht wird, bei bestimmten Gelegenheiten (z. B. Visite des Arztes etc.) mehrfach innerhalb einer Stunde. Die Pflege und Feuchthaltung der Haut ist in einer solchen Situation von essentieller Bedeutung, so daß für diese Fälle Feuchthaltemittel eine wichtige Rolle spielen. Beispiele derartiger Feuchthaltemittel sind Alkylenglykole wie beispielsweise Triethylenglykol oder Glycerin.

In einer besonders bevorzugt erfindungsgemäß zu verwendenden alkoholischen Desinfektionsmittel-Zubereitung sind beispielsweise zusätzlich zu den alkoholischen Komponenten, d. h. Ethanol und/oder Propanol sowie Butanon 0,1 bis 0,3 Gew.-% Glycerin, 0,2 bis 0,6 Gew.-% Triethylenglykol und 0,2 bis 1,0 Gew.-% Isopropylmyristat enthalten. Die Erfindung ist bezüglich der genannten Pflege- und Feuchthaltemittel-Komponenten weder auf die vorstehend beispielhaft genannten Verbindungen noch auf die für diese Verbindungen genannten Mengen beschränkt.

Zusätzlich zu den vorstehend genannten Komponenten können die erfindungsgemäß zu verwendenden alkoholischen Desinfektionsmittel-Zubereitungen noch Wasser, für derartige Zubereitungen übliche Konservierungsstoffe, Farbstoffe, Duftstoffe und/oder andere übliche Hilfsstoffe enthalten. Diese tragen in der Mehrzahl der Fälle nicht zur desinfizierenden Wirkung der Komponente bei, sondern dienen der Lagerbarkeit sowie ästhetischen Zwecken. Es ist jedoch auch möglich, solche Komponenten zu verwenden, die sowohl eine (desinfizierende, pflegende, feuchthaltende, konservierende usw.) Wirkung entfalten und dabei gleichzeitig für eine bestimmte Farbe und/oder einen angenehmen Duft sorgen.

Erfindungsgemäß lassen sich die vorstehend beschriebenen alkoholischen Desinfektionsmittel-Zubereitungen zur Hände- und Hautdesinfektion mit dem Ziel einer Inaktivierung behüllter und unbehüllter Viren wie beispielsweise von Polioviren, jedoch auch mit dem Ziel einer Inaktivierung behüllter Viren wie beispielsweise HBV und HIV verwenden, wobei eine Eignung sowohl für die chirurgische als auch für die hygienische Händedesinfektion angestrebt war und auch erreicht werden konnte.

Die erfindungsgemäß zu verwendende alkoholische Desinfektionsmittel-Zubereitung hat einen angenehmen, leichten Geruch und läßt sich - was für die praktische Applikation wichtig ist - leicht auf der Haut verreiben. Nach der Applikation bleibt kein störender Film auf der Haut zurück. Auch bei mehrmaliger Applikation, ohne daß die Haut/Hand zwischendurch gewaschen wird, entsteht kein klebriges und damit unangenehmes Hautgefühl. Dies ist in der Praxis wichtig für die Arbeit des Arztes bzw. Pflegepersonals auf Intensiv- oder Infektionsabteilungen, da dort die Hände vor jedem Wechsel zu einem anderen Patienten desinfiziert werden müssen, ohne daß eine Verschmutzung der Hände auftritt, die zum Waschen der Hände vor der Desinfektion Anlaß gibt.

Wegen seiner überraschend kurzen Einwirkzeit/Zeit zur Reduktion des Keimtiters ist die Verwendung des Mittels auch für die Desinfektion der Haut vor chirurgischen Eingriffen oder vor dem Setzen von Spritzen geeignet.

Die Erfindung wird durch die folgenden Beispiele näher erläutert.

### Beispiel 1

### Rezepturen

Durch Zusammenmischen der nachfolgend genannten Komponenten in einer üblichen geschlossenen Rührapparatur wurden die nachfolgenden erfindungsgemäß zu verwendenden alkoholischen Desinfektionsmittel-Zubereitungen hergestellt.

### Zubereitung 1:

| | |
|---|---|
| Ethanol: | 90 % |
| Butanon: | 0,9 % |
| Aqua purificata: | ad 100 % |

### Zubereitung 2:

| | |
|---|---|
| Ethanol: | 90 % |
| Butanon: | 0,9 % |
| Chlorhexidin: | 0,2 % |
| Aqua purificata: | ad 100 % |

### Zubereitung 3:

| | |
|---|---|
| Ethanol: | 95 bis 98 % |
| Butanon: | 0,5 bis 2,0 % |
| Chlorhexidindigluconat: | 0,05 bis 0,5 % |
| Octyloctanoat: | 0,05 bis 1,5 % |
| Triethylenglykol: | 0,02 bis 1,75 % |
| Glycerin: | 0,05 bis 0,75 % |
| Farbstoff, Konservierungsstoff, Parfüm nach Belieben Aqua purificata: | ad 100 %. |

### Zubereitung 4:

| | |
|---|---|
| Ethanol: | 90,0 % |
| Butanon: | 0,9 % |
| Chlorhexidindigluconat: | 0,2 % |
| Glycerin: | 0,2 % |
| Isostearylhexanoat: | 0,7 % |
| Farbstoff, Konservierungsstoff, Parfüm nach Belieben Aqua purificata: | ad 100 % |

### Vergleichszubereitung:

| | |
|---|---|
| Ethanol: | 90 % |
| Aqua purificata: | ad 100 %. |

### Beispiel 2

### Bestimmung der Reduktionsfaktoren des Poliotiters im Suspensionsversuch nach DVV

Die Bestimmung wurde nach den Richtlinien der DVV (Deutsche Vereinigung zur Bekämpfung von Viruskrankheiten) in standardisierter Weise durchgeführt. Dabei ergaben sich die aus der nachfolgenden Tabelle 1 ersichtlichen Reduktionsfaktoren bei Einwirkzeiten der Desinfektionsmittel-Zubereitungen von 30 s, 60 s und 120 s.

**Tabelle 1**

| Reduktionsfaktoren des Poliotiters im Suspensionsversuch nach DVV | | | |
|---|---|---|---|
| | Einwirkzeit | | |
| Zubereitung | 30 s | 60 s | 120 s |
| Vergleich | 2,75 | 5,62 | 6,00 |
| 1 | 5,00 | 5,62 | 6,00 |
| 2 | 4,57 | 5,75 | 5,75 |
| 3 | 4,87 | >5,00 | >5,00 |
| 4 | >5,00 | >5,00 | >5,00 |

Wie sich aus Tabelle 1 ergibt, ist die Wirksamkeit der erfindungsgemäß zu verwendenden Zubereitungen bei kurzen Einwirkzeiten erheblich der Wirksamkeit von Zubereitungen, die nur Ethanol enthalten, überlegen. Bereits ein Zusatz von Butanon, der 1 % des Ethanolgehalts beträgt (Zubereitung 1), führt zu einer erheblichen Steigerung der Wirksamkeit der erfindungsgemäß zu verwendenden Desinfektionsmittel-Zubereitung gegenüber einer ethanolischwäßrigen Lösung.

Der Zusatz eines Remanenzwirkstoffs (Zubereitung 2) sowie eines Remanenzwirkstoffs zusammen mit bestimmten Pflege- und Feuchthaltekomponenten (Zubereitungen 3 und 4) führt zu keiner Einbuße in bezug auf die Wirksamkeit gegenüber Polioviren. Die Wirksamkeit der Zubereitung 4 ist der aller anderen Zubereitungen bei kurzer Einwirkzeit überlegen.

### Beispiel 3

### Bestimmung der Inaktivierung von Hepatitis B-Viren im DNA Polymerase Test

Die Bestimmung der Inaktivierung von HB-Viren wurde indirekt durchgeführt. Dafür sind vom BGH drei Testmethoden zugelassen worden, deren Ergebnisse bei der Zulassung von Arzneimitteln als Nachweis einer Inaktivierung von Hepatitis B-Viren gelten. Es sind dies der Oberflächenantigen-Test, der MADT-Test und der DNA-Polymerase-Test. Zur Bestimmung der Wirksamkeit des Mittels wurde der DNA-Polymerasetest gewählt, da dieser Test eine quantitative Aussage über den Anteil etwaig noch infektiöser Restmengen an Viren erlaubt und die Zerstörung der Viren-DNA mißt. Der Test wurde mit den erfindungsgemäß zu verwendenden Zubereitungen durchgeführt.

**Tabelle 2**

| **HBV-inaktivierende Eigenschaften einer erfindungsgemäß zu verwendenden Zubereitung im DNS-Polymerase-Test bei 20 °C. Angegeben sind die Counts pro Minute (cpm)** | | | | | | | | | |
|---|---|---|---|---|---|---|---|---|---|
| Einwirkzeit (min) | Dane-Partikel-Kontrollen | | | Desinfektionsmittel-Kontrollen | | | erfindungsgemäße Zubereitung (80 %) | | |
| | I | II | III | I | II | III | I | II | III |
| 0,5 | 4781 | 4691 | 4909 | - | - | - | 141 | n.d. | n.d. |
| 1,0 | n.d. | n.d. | n.d. | - | - | - | 10 | 7 | 11 |
| 2,0 | n.d. | n.d. | n.d. | - | - | - | 12 | 13 | 8 |
| 5,0 | n.d. | n.d. | n.d. | - | - | - | 11 | n.d. | n.d. |
| 10,0 | 4699 | 5007 | 4918 | 13 | 12 | 9 | 9 | n.d. | n.d |
| Anmerkung: n.d. = nicht durchgeführt | | | | | | | | | |

Wie aus der Tabelle ersichtlich, ist die notwendige Zeit bis zur vollständigen Zerstörung der vitalen DNA sehr viel kürzer als nach dem Stand der Technik. Die Nachweisgrenze des DNA-Polymerase-Tests ist nach einer Minute erreicht, da die erfindungsgemäß zu verwendende Zubereitung bereits ohne DNA-Partikel bis zu 13 cpm auslöst. Bekannte Zubereitungen auf Basis von maximal 80 g Ethanol, Isopropanol oder n-Propanol benötigten mindestens 3 min, in einem Fall eines Mittels nach Stand der Technik 5 bis 15 min, um die Hepatitis B-Viren zu zerstören.

### Beispiel 4

Die Wirksamkeit der erfindungsgemäß zu verwendenden Desinfektionsmittel-Zubereitungen wurde in den standardisierten Prüfverfahren der DGHM gegen Bakterien und Pilze und in den praxisnahen Versuchen getestet. Dabei zeigte sich, daß die Zubereitung 4 bereits nach 30 s im Suspensionsversuch eine vollständige Abtötung aller Prüfkeime erreichte. Außerdem war die Zubereitung 4 bereits bei der chirurgischen Händedesinfektion nach DGHM in 2 Minuten so wirksam wie das Referenzverfahren nach 5 Minuten. Bei gleich guter Bakterizidie wie die besten zur Zeit im Handel befindlichen Händedesinfektionsmittel war das Produkt sehr gut hautverträglich und bot zusätzlich Schutz gegen Polioviren und andere behüllte Viren, jedoch auch gegen unbehüllte Viren (HBV, HIV) in 30 s nach DVV.

## Patentansprüche

1. Verwendung einer alkoholischen Desinfektionsmittel-Zubereitung mit einem Gehalt an Ethanol und/oder Methanol von mindestens 80 Gew.-% zur Inaktivierung von Viren bei der Hände- und Hautdesinfektion, dadurch gekennzeichnet, daß die Desinfektionsmittelzubereitung Butanon enthält.

2. Verwendung nach Anspruch 1, bei dem die Desinfektionsmittelzubereitung mindestens 0,3 Gew.-% Butanon enthält.

3. Verwendung nach einem der Ansprüche 1 oder 2, bei der der Butanongehalt in der Desinfektionsmittelzubereitung zwischen 0,3 und 2,5 Gew.-%, vorzugsweise zwischen 0,8 und 1,3 Gew.-% liegt.

4. Verwendung nach einem der Ansprüche 1 bis 3, bei der in der Desinfektionsmittelzubereitung zusätzlich ein Remanenzwirkstoff enthalten ist.

5. Verwendung nach Anspruch 4, bei der der Remanenzwirkstoff aus der Gruppe Chlorhexidin und Benzalkoniumchlorid und deren Derivate stammt und bevorzugt Chlorhexidindiglukonat ist.

6. Verwendung nach einem der Ansprüche 4 oder 5, bei der die Menge an Remanenzwirkstoff in der Desinfektionsmittelzubereitung 0,025 bis 1,0 Gew.-% beträgt.

7. Verwendung nach einem der Ansprüche 1 bis 6, bei der in der Desinfektionsmittelzubereitung zusätzlich als Pflegekomponente ein Ester einer langkettigen Fettsäure enthalten ist.

8. Verwendung nach Anspruch 7, bei der als Pflegekomponente ein C₁ bis C₈-Alkylester einer Fettsäure mit 8 bis 18 Kohlenstoffatomen, vorzugsweise ein C₁ bis C₄-Alkylester einer Fettsäure mit 12 bis 18 Kohlenstoffatomen enthalten ist.

9. Verwendung nach einem der Ansprüche 1 bis 8, bei der in der Desinfektionsmittelzubereitung zusätzlich als Feuchthaltemittel ein Alkylenglykol oder Glycerin enthalten sind.

10. Verwendung nach Anspruch 9, bei der als Feuchthaltemittel Triethylenglykol in der Desinfektionsmittelzubereitung enthalten ist.

11. Verwendung nach einem der Ansprüche 1 bis 10, bei der in der Desinfektionsmittelzubereitung zusätzlich zu den alkoholischen Komponenten und Butanon 0,1 bis 0,3 Gew.-% Glycerin, 0,2 bis 0,6 Gew.-% Triethylenglykol und 0,2 bis 1,0 Gew.-% Isopropylmyristat enthalten sind.

12. Verwendung nach einem der Ansprüche 1 bis 11, bei der in der Desinfektionsmittelzubereitung zusätzlich Wasser, Konservierungsstoffe, Farbstoffe, Duftstoffe und/oder andere übliche Hilfsstoffe enthalten sind.

13. Verwendung nach einem der Ansprüche 1 bis 12, zur Inaktivierung von unbehüllten Viren, insbesondere Polioviren.

14. Verwendung nach einem der Ansprüche 1 bis 12, zur Inaktivierung von behüllten Viren, insbesondere Hepatitis B-Viren und HIV-Viren.

## Claims

1. Use of an alcohol-based disinfectant preparation having a content of ethanol and/or methanol of at least 80 % by weight for inactivating viruses during disinfecting the hands and the skin, characterized in that the disinfectant preparation comprises butanone.

2. Use according to claim 1, characterized in that the disinfectant preparation has a content of butanone of at least 0,3 % by weight.

3. Use according to claim 1 or claim 2, characterized in that the disinfectant preparation comprises butanone within a range of from 0,3 to 2,5 % by weight, preferably of from 0,8 to 1,3 % by weight.

4. Use according to any of the claims 1 to 3, characterized in that the disinfectant preparation further contains a substance providing remanence.

5. Use according to claim 4 wherein said substance providing remanence is selected from chlorhexidine and benzalconiumchloride and preferably is chlorhexidine gluconate.

6. Use according to claim 4 or 5, wherein the amount of said substance providing remanence in the disinfectant preparation is 0,025 to 1,0 % by weight.

7. Use according to any of the claims 1 to 6, characterized in that the disinfectant preparation further contains an ester of a long chain fatty acid as a component providing care to the skin.

8. Use according to claim 7, characterized in that the disinfectant preparation contains a C₁ to C₈ alkylester of a fatty acid having 8 to 18 carbon atoms, preferably a C₁ to C₄ alkylester of a fatty acid having 12 to 18 carbon atoms, as said component providing care to the skin.

9. Use according to any of the claims 1 to 8, characterized in that the disinfectant preparation further contains an alkylene glycol or glycerol as a moisturizer.

10. Use according to claim 9, characterized in that the disinfectant preparation contains triethylene glycol as the moisturizer.

11. Use according to any of the claims 1 to 10, characterized in that the disinfectant preparation contains, in addition to the alcoholic components and to butanone, 0,1 to 0,3 % by weight of glycerol, 0,2 to 0,6 % by weight of triethylene glycol and 0,2 to 1,0 % by weight of isopropyl myristate.

12. Use according to any of the claims 1 to 11, characterized in that the disinfectant preparation further contains water, preservatives, colouring agents, flavours and/or other adjuvants.

13. Use according to any of the claims 1 to 12 for inactivating non enveloped viruses, in particular polio viruses.

14. Use according to any of the claims 1 to 12 for inactivating enveloped viruses, in particular hepatitis B-viruses and HI viruses.

## Revendications

1. Utilisation d'une préparation alcoolique d'agent désinfectant présentant une concentration en éthanol et/ou en méthanol d'au moins 80 % en poids, pour l'inactivation de virus lors de la désinfection des mains et de la peau, caractérisé en ce que la préparation d'agent désinfectant renferme de la butanone.

2. Utilisation selon la revendication 1, dans laquelle la préparation d'agent désinfectant renferme au moins 0,3 % en poids de butanone.

3. Utilisation selon la revendication 1 ou 2, dans laquelle la concentration en butanone dans la préparation d'agent désinfectant est comprise entre 0,3 et 2,5 % en poids, de préférence entre 0,8 et 1,3 % en poids.

4. Utilisation selon une des revendications 1 à 3, dans laquelle la préparation d'agent désinfectant renferme en plus une substance active de rémanence.

5. Utilisation selon la revendication 4, dans laquelle la substance active de rémanence appartient au groupe de la chlorhexidine et du chlorure de benzalkonium et des dérivés de ceux-ci et est de préférence le digluconate de chlorhexidine.

6. Utilisation selon une des revendications 4 ou 5, dans laquelle la proportion de substance active de rémanence dans la préparation d'agent désinfectant atteint 0,025 à 1,0 % en poids.

7. Utilisation selon une des revendications 1 à 6, dans laquelle la préparation d'agent désinfectant renferme en plus comme composant soignant un ester d'un acide gras à chaîne longue.

8. Utilisation selon la revendication 7, dans laquelle la préparation d'agent désinfectant renferme comme composant soignant, un ester alkylique en C₁ à C₈ d'un acide gras comportant 8 à 18 atomes de carbone, de préférence, un ester alkylique en C₁ à C₄ d'un acide gras présentant 12 à 18 atomes de carbone.

9. Utilisation selon une des revendications 1 à 8, dans laquelle la préparation d'agent désinfectant renferme en plus comme humidifiant, un alkylènelglycol ou de la glycérine.

10. Utilisation selon la revendication 9, dans laquelle la préparation d'agent désinfectant renferme comme humidifiant, du triéthylèneglycol.

11. Utilisation selon une des revendications 1 à 10, dans laquelle la préparation d'agent désinfectant renferme en plus des composants alcooliques et de la butanone, 0,1 à 0,3 % en poids de glycérine, 0,2 à 0,6 % en poids de triéthylèneglycol et 0,2 à 1,0 % en poids d'isopropylmyristate.

12. Utilisation selon une des revendications 1 à 11, dans laquelle la préparation d'agent désinfectant renferme en plus de l'eau, des conservateurs, des colorants, des parfums et/ou d'autres adjuvants usuels.

13. Utilisation selon une des revendications 1 à 12 pour l'inactivation des virus non enveloppés, en particulier les poliovirus.

14. Utilisation selon une des revendications 1 à 12 pour l'inactivation des virus enveloppés, en particulier les virus de l'hépatite B et les virus HIV.
